# FASCICULE DE BREVET EUROPEEN

(11) **EP 0 680 479 B1**
(45) Date de publication et mention de la délivrance du brevet: **17.03.1999**
(21) Numéro de dépôt: 94903710.5
(22) Date de dépôt: 19.01.1994
(51) Int. Cl.: C07D 473/08

(54) **PROCEDE DE PREPARATION DE BAMIFYLLINE ET D'ETAMIPHYLLINE**
HERSTELLUNGUNGSVERFAHREN VON BAMIFYLLIN UND ETAMIPHYLLIN
METHOD FOR PREPARING BAMIFYLLINE AND ETAMIPHYLLINE

(30) Priorité: 21.01.1993 BE 9300059
(43) Date de publication de la demande: 08.11.1995
(73) Titulaire: S.A. NYCOMED CHRISTIAENS N.V., B-1080 Bruxelles (BE)
(72) Inventeur: THYRION, Fernand, B-1140 Bruxelles (BE); YANG, Hong, B-1348 Louvain-la-Neuve (BE); PARMANTIER, Michel, B-1150 Bruxelles (BE)
(74) Mandataire: Powis de Tenbossche, Roland
(86) Numéro de dépôt international: BE9400006
(87) Numéro de publication internationale: WO9417064

(56) Documents cités:
- BE-A- 602 888
- BE-A- 883 654
- FR-M- 1 124
- FR-M- 4 291

## Description

La présente invention a pour objet un procédé de préparation de Bamifylline ou d'étamiphylline de formule 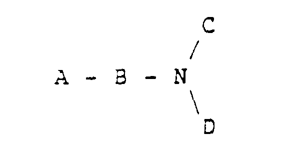 dans laquelle
A est un cycle 8-benzylthéophylline ou théophylline
B est un groupe hydrocarboné à 2 atomes de carbone,
C et D sont des groupes hydrocarbonés identiques ou différents, portant éventuellement un ou plusieurs groupes hydroxyle ou d'un sel d'un tel composé. Dans ce procédé on fait réagir la 8-benzylthéophylline ou théophylline avec un excès molaire d'une substance X₁ - B - X₂ avec X₁ et X₂ des groupes électrophiles en présence d'un excès molaire d'une amine de formule : 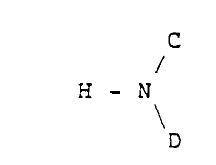 et éventuellement on convertit le composé formé en un sel de celui-ci.

On connaît par le brevet belge BE-A-602 888 la préparation de la bamifylline, à savoir un composé de formule II.

Selon ce brevet, on prépare la bamifylline en faisant réagir dans un premier stade la 8-benzylthéophylline avec du 1,2-dichloréthane ou du 1,2-dibrométhane pour obtenir de la 7-(β-chloro ou β-bromo-éthyl)-8-benzylthéophylline (Formule I).

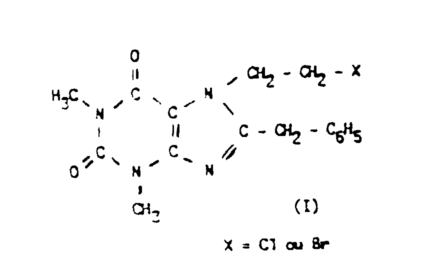

Dans un second stade, le produit (I) est mis en réaction avec le 2-éthylamino-éthanol en présence de carbonate de sodium pour former la bamifylline (II) avec un rendement de 57 à 65% selon le schéma suivant :

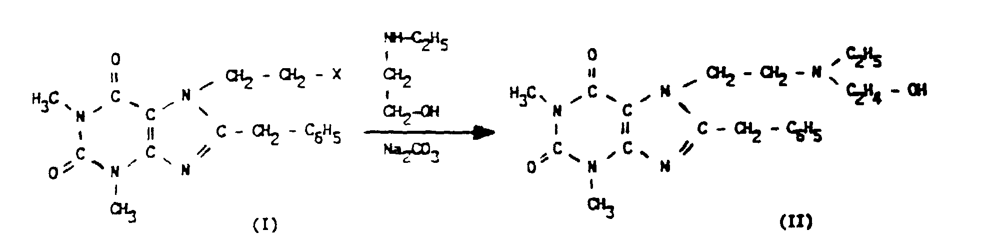

Cette réaction donne lieu à la formation du dérivé 7-vinyl-8 benzylthéophylline (III) comme produit secondaire en quantité importante (25 à 43%).

Par le brevet belge BE-A-883 654, on connaît la préparation de bamifylline en un seul stade avec un bon rendement en chauffant au reflux un mélange réactionnel constitué de 8-benzylthéophylline, N-éthylamino-éthanol, carbonate de sodium et 1,2-dichloréthane.

Ce procédé. qui permet d'éviter la formation du sous-produit vinylique (III), 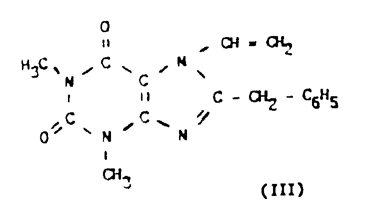 présente cependant les inconvénients d'un temps de réaction très long (52 heures) et de la formation d'environ 7% du dérivé 7-chloréthyl-8-benzylthéophylline (I) comme sous-produit. La température de réaction dans le procédé selon ce brevet est de 80°C.

Dans le procédé suivant l'invention, on effectue la réaction à une température supérieure à 90°C et sous une pression supérieure à 10⁵ Pa.

On a remarqué que le procédé suivant l'invention permettait d'accroître la vitesse de conversion du cycle nucléophile, et de façon tout à fait inattendue d'assurer une exellente sélectivité de la conversion de la 8-benzylthéophylline ou théophylline en bamifylline ou en étamiphylline.

Si l'homme du métier pouvait s'attendre à ce que la vitesse de la réaction 8-benzylthéophylline + N-éthylamino-éthanol + 1,2-dichloroéthane à 90°C soit supérieure à la vitesse de cette réaction à 80°C, l'homme du métier ne pouvait s'attendre à ce que le procédé selon l'invention permette d'assurer encore une sélectivité de la réaction en bamifylline supérieure à 90%. En effet, l'homme du métier sait qu'en accroissant la température d'une réaction, on favorise non seulement la réaction désirée, mais également des réactions secondaires donnant des sous-produits indésirés.

Ainsi de façon tout à fait inattendue, on a remarqué qu'en appliquant le procédé suivant l'invention à la fabrication de bamifylline (8-benzylthéophylline + N-éthylamino-éthanol + 1,2-dichloroéthane), il a été possible d'obtenir une conversion supérieure à 99%, de 8-benzylthéophylline apres un temps de réaction de 2 à 5 heures (au lieu de 52 heures pour le procédé selon BE-A-883 654) et un rendement molaire en bamifylline par rapport au nombre de moles de 8-benzylthéophylline supérieur à 90%, voire plus (> 95%).

De façon avantageuse, on effectue la réaction à une température supérieure à 110°C, de préférence à 125°C.

Selon une forme de réalisation du procédé suivant l'invention, on fait réagir un cycle de 8-benzylthéophylline ou théophylline avec une substance X₁ - B - X₂ dans laquelle X₁ et X₂ sont des atomes d'halogène, 3 est un groupe hydrocarboné contenant 2 atomes de carbone en présence d'une amine 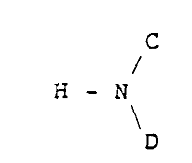 dans laquelle C et D sont choisis parmi les groupes ethyle contenant 2 atomes de carbone et contenant éventuellement jusque 2 groupes hydroxyle.

Selon une particularité de cette forme de réalisation, on fait réagir de la 8-benzylthéophylline avec un dihalogénure d'alkyle, en particulier du dichloroéthane, en présence d'un alkylaminoalcanol.

La réaction est avantageusement effectuée dans un milieu polaire en présence d'une base faible telle que Na₂CO₃, Na₃PO₄ ou un mélange de ceux-ci.

Dans des formes de réalisations préférées du procédé selon l'invention, on fait réagir la 8-benzylthéophylline ou théophylline avec un excès molaire de substance X₁ - B - X₂ de plus de 100%.

Le rapport molaire X₁ - B - X₂- 8/- benzylthéophylline ou théophylline est par exemple supérieur à 5 mais est de préférence supérieur ou sensiblement égal à 10.

Des exemples particuliers de composés de formule X₁ - B - X₂ sont le 1,2-dichloroéthane, le 1-chloro, 2-bromoéthane, le 1,2-dibromoéthane. Les composés chlorés sont toutefois préférés vu leur meilleure sélectivité.

La quantité d'amine de formule : 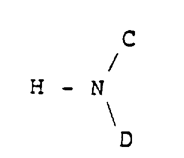 utilisée dans la réaction du procédé suivant l'invention est quant à elle avantageusement choisie de telle manière que le rapport molaire : 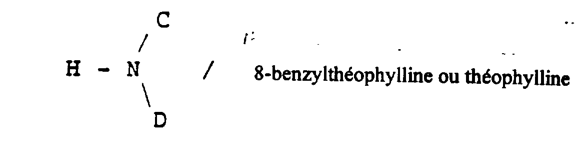 soit supérieur à 1,2, de préférence à environ 1,5 au début de la réaction.

La réaction du procédé suivant l'invention peut être réalisée dans un solvant, par exemple un solvant polaire ou apolaire. Ce solvant, qui peut être un des réactifs de la réaction (cf. ex. dichloroéthane), est avantageusement un éther, un solvant chloré, un alcane, un alcanol tel qu'éthanol, propanol, isopropanol, n-pentanol, n-butanol, des aromatiques tels que du xylène, du dioxanne, ou un mélange de ceux-ci. En particulier le solvant utilisé sera le dioxanne ou un mélange contenant au moins 25%, de préférence 50% de dioxanne, le restant dudit mélange étant constitué d'un réactif de la réaction agissant également en tant que solvant, un alcane, un éther, un solvant chloré, un aromatique un alcanol, du xylène ou un mélange de ceux-ci.

On notera que le dioxanne semble être un composé favorisant la sélectivité de la conversion de la 8-benzylthéophylline ou théophylline en bamifylline ou en étamiphylline.

La présente invention a donc encore pour objet l'utilisation du dioxanne en tant que milieu pour la conversion de 8-benzylthéophylline ou théophylline en bamifylline ou en étamiphylline et ce quelle que soit la température ou la pression de la réaction.

D'autres particularités et détails de l'invention ressortiront de la description détaillée suivante dans laquelle il est fait référence à des exemples de préparation de dérivés de Xanthine.

### Exemple 1.-

On a chauffé à 125°C pendant 3 heures dans un autoclave fermé muni d'une soupape pour y maintenir une pression constante de + 4 bars (4 10⁵ Pa), le mélange suivant :

| | | |
|---|---|---|
| 15 g | de 8-benzylthéophylline | (0,055 mole) |
| 7,6 g | de 2-éthylamino-éthanol | (0,085 mole) |
| 45 ml | de 1,2-dichloréthane | (0,57 mole) |
| 11 g | de Na₂CO₃ anhydre | (0,104 mole) |

Par contrôle de la réaction par chromatographie en phase liquide (HPLC) ou chromatographie en couche mince, on a remarqué que la transformation de la 8-benzylthéophylline était totale et que les produits de la réaction du cycle nucléophile comprenait 98% minimum de bamifylline et 2% maximum de 7-chloroéthyl-8-benzylthéophylline. Le milieu réactionnel a ensuite été traité à l'eau pour dissoudre les sels minéraux. la phase organique a alors été extraite avec de l'acide chlorhydrique puis éliminée.

La phase aqueuse acide a été neutralisée avec du carbonate de sodium et le [7-(N-éthyl-N-(β- hydroxyéthyl)aminoéthyl)]-8-benzylthéophylline en a été extrait par un hydrocarbure aliphatique halogéné tel que le dichlorométhane.

Après évaporation à sec du dichlorométhane, la base a été dissoute dans un alcanol tel que le méthanol puis transformée en chlorhydrate par addition d'acide chlorhydrique. Le chlorhydrate obtenu a été purifié par cristallisation dans du méthanol.

Le chlorhydrate de bamifylline de point de fusion 185-188°C a été obtenu avec un rendement réel de 93%.

### Exemple 2.-

On a chauffé à 125°C pendant 5 heures dans les conditions décrites dans l'exemple 1 :

| | | |
|---|---|---|
| 10 g | de 8-benzylthéophylline | (0,037 mole) |
| 5 g | de 2-éthylamino-éthanol | (0,056 mole) |
| 30 ml | de xylène | |
| 30 ml | de 1,2-dichloréthane | (0,38 mole) |
| 7,3 g | de Na₂CO₃ anhydre | (0,069 mole) |

On a contrôlé la réaction par HPLC et celle-ci indique l'absence de 8-benzylthéophylline et la présence de 99,8% de bamifylline et 0,2% de 7-chloroéthyl-8-benzylthéophylline.

Les solvants ont été éliminés sous vide et le résidu a été repris par de l'eau. On a procédé à l'isolement du chlorhydrate de bamifylline comme décrit dans l'exemple 1.

### Exemple 3.-

On a chauffé à 125°C pendant 3 heures dans les conditions décrites dans l'exemple 1 :

| | | |
|---|---|---|
| 10 g | de 8-benzylthéophylline | (0,037 mole) |
| 5 g | de 2-éthylamino-éthanol | (0,056 mole) |
| 20 ml | de n-pentanol | |
| 40 ml | de 1,2-dichloréthane | (0,51 mole) |
| 7,3 g | de Na₂CO₃ anhydre | (0,069 mole) |

On a contrôlé la réaction par chromatographie HPLC et celle-ci a indiqué l'absence de 8-benzylthéophylline et la présence de 99,4% de bamifylline et 0,6 % de 7-chloroéthyl-8-benzylthéophylline.

Les solvants ont été éliminés sous vide et le résidu a été repris par de l'eau. On a procédé à l'isolement du chlorhydrate de bamifylline comme décrit dans l'exemple 1.

### Exemple 4.-

On a répété l'exemple 2 si ce n'est qu'on a utilisé du dioxanne en tant que solvant. La conversion de la 8-benzylthéophylline était de 100% en bamifylline.

### Exemple 5.-

On a répété l'exemple 1 si ce n'est que l'on a effectué la réaction à une température de 146°C au lieu de 125°C.

Le rendement en bamifylline observé par HPLC était de 97,7% après 2 heures de réaction.

Le tableau suivant reprend les caractéristiques des exemples 1 à 5 décrits ci-avant qui sont relatifs à la transformation de 8-benzylthéophylline.

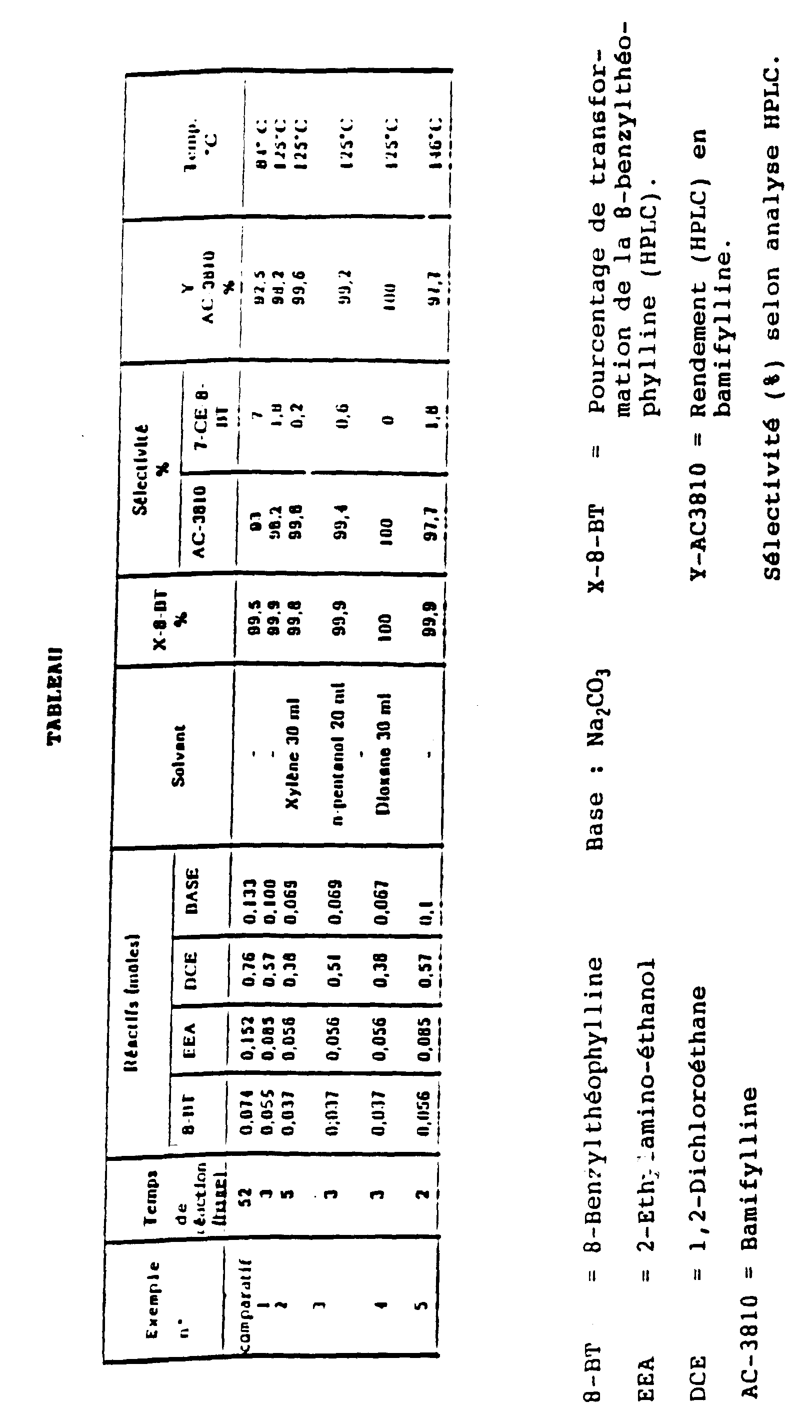

### Exemple 6.-

Dans cet exemple, on a appliqué le procédé suivant l'invention à la conversion de la théophylline en étamiphylline selon le processus réactionnel suivant.

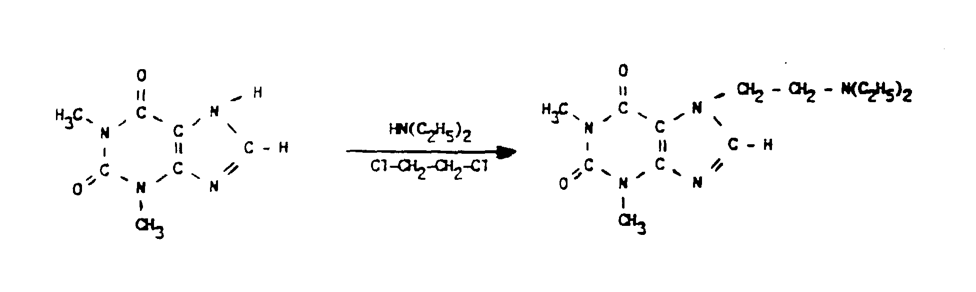

On a fait réagir dans cet exemple 10 g de théophylline (0,056 mole), 8,8 ml de diéthylamine (0,085 mole), 45 ml de 1,2-dichloroéthane (0,57 mole) et 10,6 g de Na₂CO₃ (0,1 mole) dans un autoclave à une température de 125°C pendant 3 heures. La température de réaction était supérieure à la température de reflux du 1,2-dichloroéthane de sorte que la réaction a été effectuée sous une pression supérieure à 10⁵ Pa.

Par chromatographie HPLC, on a déterminé que le taux de conversion de la théophylline était de 97,3%.

Le milieu réactionnel a ensuite été traité à l'eau et la phase organique a été lavée à l'aide d'une solution d'acide chlorhydrique.

Après séparation de la phase organique, le pH de la solution aqueuse-acide a été ajusté à 9,5-10 par ajout de carbonate de soude, après quoi, celle-ci a été soumise à une extraction en plusieurs étapes au dichorométhane. Après séchage, on a obtenu un résidu solide d'étamiphylline. Après une étape de cristallisation, le point de fusion du produit était de 72°C, température correspondant au point de fusion de l'étamiphylline.

La sélectivité de la réaction en étamiphylline était de 96,1%.

### Exemple 7. -

On a répété l'exemple 6 si ce n'est qu'on a effectué la réaction à une température de 112°C. Le taux de conversion de la théophylline était de 96,9% tandis que la sélectivité en étamiphylline était de 94,2%.

### Exemple 8 (comparatif).-

On a répété l'exemple 6 si ce n'est qu'on a effectué la réaction dans un réacteur ouvert surmonté d'un condenseur et que la température de réaction était de 75°C.

Un taux de conversion de 95% de la théophylline n'a pu être obtenu qu'après 14 heures de réaction. La sélectivité en étamiphylline était de 91,9%.

Le tableau suivant reprend les caractéristiques des exemples 6 à 8 décrits ci-avant.

| Exemple | Temps de réaction (heures) | Température °C | Taux de conversion de la théophylline (%) | Sélectivité en étamiphylline (%) |
|---|---|---|---|---|
| 6 | 3 | 125 | 97,3 | 96,1 |
| 7 | 3 | 112 | 96,9 | 94,2 |
| 8 | 14 | 75 | 95,2 | 91,9 |

## Revendications

1. Procédé de préparation d'un composé choisi parmi la bamifylline et l'étamiphylline dans lequel on fait réagir en présence d'une base faible, un mélange contenant :
a) de la 8-benzylthéophylline ou de la théophylline ;
b) un excès molaire d'un dihalogénure d'alkyle avec 2 atomes de carbone ;
c) un excès molaire d'une amine de formule : 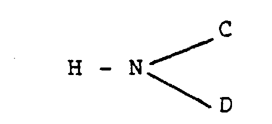 avec C et D des groupes choisis parmi les groupes alkyle contenant 2 atomes de carbone et contenant éventuellement jusque 2 groupes hydroxyle, ladite réaction étant opérée à une température supérieure à 90°C et sous une pression supérieure à 10⁵ Pa.

2. Procédé suivant la revendication 1, caractérisé en ce qu'on effectue la réaction à une température supérieure à 110°C.

3. Procédé suivant la revendication 1, caractérisé en ce qu'on effectue la réaction à une température de 125°C.

4. Procédé suivant l'une quelconque des revendications 1 à 3, caractérisé en ce que le dihalogénure d'alkyle est choisi parmi le 1,2-dichloroéthane, le 1-chloro 2-bromoéthane, le 1,2-dibromoéthane.

5. Procédé suivant la revendication 4, caractérisé en ce que le dihalogénure d'alkyle est le 1,2-dichloroéthane.

6. Procédé suivant l'une quelconque des revendications précédentes, caractérisé en ce qu'on effectue la réaction en présence d'un alkylamino-alcanol.

7. Procédé suivant l'une quelconque des revendications précédentes, caractérisé en ce qu'on effectue la réaction avec un excès molaire de 100% de dihalogénure d'alkyle, le rapport molaire dihalogénrue d'alkyle/ 8-benzylthéophylline ou théophylline étant avantageusement supérieur à 5, de préférence à 10.

8. Procédé suivant l'une quelconque des revendications précédentes, caractérisé en ce qu'on effectue la réaction avec un excès d'amine, le rapport molaire amine/ 8-benzylthéophylline ou théophylline étant supérieur à 1, 2.

9. Procédé suivant l'une quelconque des revendications précédentes, caractérisé en ce qu'on effectue la réaction dans un solvant de préférence un alcanolxylène, du dioxanne, un éther, un solvant chloré, un alcane ou un mélange de ceux-ci.

10. Procédé suivant l'une quelconque des revendications précédentes, caractérisé en ce qu'on utilise à titre de base faible du Na₂CO₃, du Na₃PO₄ ou un mélange de ceux-ci.

## Patentansprüche

1. Verfahren zur Herstellung einer Verbindung, die unter Bamifyllin und Etamiphyllin ausgewählt ist, bei dem man in Gegenwart einer schwachen Base ein Gemisch umsetzt, das enthält:
a) 8-Benzyltheophyllin oder Theophyllin,
b) einen molaren Überschuß eines Alkyldihalogenids mit 2 Kohlenstoffatomen,
c) einen molaren Überschuß eines Amins der Formel: 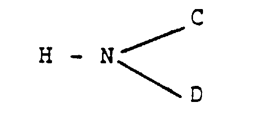 mit Gruppen C und D, die unter den Alkylgruppen mit 2 Kohlenstoffatomen und gegebenenfalls mit bis zu 2 Hydroxygruppen ausgewählt sind, wobei besagte Reaktion bei einer Temperatur, die höher als 90 °C ist, und unter einem Druck, der höher als 10⁵ Pa ist, ausgeführt wird.

2. Verfahren gemäß Anspruch 1, dadurch gekennzeichnet, daß man die Reaktion bei einer Temperatur, die höher als 110 °C ist, ausführt.

3. Verfahren gemäß Anspruch 1, dadurch gekennzeichnet, daß man die Reaktion bei einer Temperatur von 125 °C ausführt.

4. Verfahren gemäß einem der Ansprüche 1 bis 3, dadurch gekennzeichnet, daß das Alkyldihalogenid unter 1,2-Dichlorethan, 1-Chlor-2-bromethan, 1,2-Dibromethan ausgewählt ist.

5. Verfahren gemäß Anspruch 4, dadurch gekennzeichnet, daß das Alkyldihalogenid 1,2-Dichlorethan ist.

6. Verfahren gemäß einem der vorhergehenden Ansprüche, dadurch gekennzeichnet, daß man die Reaktion in Gegenwart eines Alkylaminoalkanols ausführt.

7. Verfahren gemäß einem der vorhergehenden Ansprüche, dadurch gekennzeichnet, daß man die Reaktion mit einem molaren Überschuß von 100% an Alkyldihalogenid ausführt, wobei das Molverhältnis Alkyldihalogenid / 8-Benzyltheophyllin oder Theophyllin vorteilhafterweise größer als 5, vorzugsweise größer als 10 ist.

8. Verfahren gemäß einem der vorhergehenden Ansprüche, dadurch gekennzeichnet, daß man die Reaktion mit einem Überschuß an Amin ausführt, wobei das Molverhältnis Amin / 8-Benzyltheophyllin oder Theophyllin größer als 1,2 ist.

9. Verfahren gemäß einem der vorhergehenden Ansprüche, dadurch gekennzeichnet, daß man die Reaktion in einem Lösungsmittel, vorzugsweise einem Alkanolxylol, Dioxan, einem Ether, einem chlorierten Lösungsmittel, einem Alkan oder einem Gemisch von diesen ausführt.

10. Verfahren gemäß einem der vorhergehenden Ansprüche, dadurch gekennzeichnet, daß man als schwache Base Na₂CO₃, Na₃PO₄ oder ein Gemisch von diesen verwendet.

## Claims

1. Process for the preparation of a compound chosen from bamifylline and etamiphylline, in which a mixture containing:
a) 8-benzyltheophylline or theophylline;
b) a molar excess of an alkyl dihalide with 2 carbon atoms;
c) a molar excess of an amine of formula: 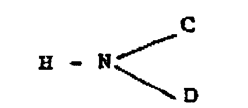 where C and D are groups chosen from alkyl groups containing 2 carbon atoms and optionally containing up to 2 hydroxyl groups, is reacted in the presence of a weak base, the said reaction being carried out at a temperature of greater than 90°C and at a pressure of greater than 10⁵ Pa.

2. Process according to claim 1, characterized in that the reaction is carried out at a temperature of greater than 110°C.

3. Process according to Claim 1, characterized in that the reaction is carried out at a temperature of 125°C.

4. Process according to any one of Claims 1 to 3, characterized in that the alkyl dihalide is chosen from 1,2-dichloroethane, 1-chloro-2-bromoethane and 1,2-dibromoethane.

5. Process according to Claim 4, characterized in that the alkyl dihalide is 1,2-dichloroethane.

6. Process according to any one of the preceding claims, characterized in that the reaction is carried out in the presence of an alkylaminoalkanol.

7. Process according to any one of the preceding claims, characterized in that the reaction is carried out with a 100% molar excess of alkyl dihalide, the alkyl dihalide/8-benzyltheophyiiine or theophylline molar ratio advantageously being greater than 5, preferably greater than 10.

8. Process according to any one of the preceding claims, characterized in that the reaction is carried out with an excess of amine, the amine/8-benzyltheophylline or theophylline molar ratio being greater than 1.2.

9. Process according to any one of the preceding claims, characterized in that the reaction is carried out in a solvent, preferably an alkanolxylene, dioxane, an ether, a chlorinated solvent, an alkane or a mixture of these solvents.

10. Process according to any one of the preceding claims, characterized in that Na₂CO₃, Na₃PO₄ or a mixture of these is used as weak base.
